# EUROPEAN PATENT SPECIFICATION

(11) **EP 2 044 408 B1**
(45) Date of publication and mention of the grant of the patent: **23.12.2009**
(21) Application number: 07734351.5
(22) Date of filing: 20.04.2007
(51) Int. Cl.: G01M 3/32

(54) **STERILE SYRINGE LEAK TESTING METHOD AND MACHINE**
LECKPRÜFVERFAHREN UND -MASCHINE FÜR STERILE SPRITZEN
PROCEDE ET DISPOSITIF DE TEST DE FUITE DE SERINGUES STERILES

(30) Priority: 21.07.2006 IT BO20060550
(43) Date of publication of application: 08.04.2009
(73) Proprietor: Bonfiglioli, Giuseppe, 44047 Sant'Agostino (IT)
(72) Inventor: Bonfiglioli, Giuseppe, 44047 Sant'Agostino (IT)
(74) Representative: Lanzoni, Luciano
(86) International application number: PCT/IB2007/001032
(87) International publication number: WO 2008/012611

(56) References cited:
- DE-A1- 3 044 550
- US-A1- 2006 129 084

## Description

### Technical Field

This invention has for an object a leak testing method for sterile syringes, in particular, syringes of the disposable type prefilled with a medicinal product.

### Background Art

Prior art methods used to manufacture and pack syringes involve filling the syringes in a sterile atmosphere and then placing them in packages ready for sale.

As is known, these syringes comprise a hollow cylindrical barrel, open at one end to enable it to be filled, and closed at the other end by a tip to which a needle is fitted and which is coupled with a cap that protects the needle. A plunger slides inside the hollow cylindrical barrel forming a seal with the latter's inside surface and delimiting, at the end opposite the tip, a sterile airtight chamber containing the medicinal product.

One of the most frequent manufacturing defects of these syringes is leakage through the chamber containing the medicinal product. This is usually due to the irregular shape of the plunger, normally of rubber, which does not adhere tightly enough to the inside surface of the syringe barrel, or to damage of the part of the syringe where the needle is connected, caused by incorrect insertion of the needle into its protective cap.

Leaking through the chamber is a very serious defect which exposes the medicinal product to the risk of contamination during the subsequent stage of packaging the syringe.

In DE3044550 a method is disclosed for testing syringes on leakage by closing one opening of the unfilled syringe and moving the plunger so that an overpressure is produced, releasing the plunger and then comparing the final position of the plunger to the start position.

### Disclosure of the Invention

The aim of this invention is to provide a sterile syringe leak testing method whereby syringes with medicinal containing chambers that are not tightly sealed can be positively detected.

This invention accordingly provides a sterile syringe leak testing method as defined in any of the appended claims.

Another aim of this invention is provide a sterile syringe leak testing machine.

### Brief Description of the Drawings

The invention will now be described with reference to the accompanying drawings which illustrate a preferred non-restricting embodiment of it and in which:
- Figures 1 and 2 are, respectively, a perspective view and a front view of a sterile syringe to be tested using the method according to this invention;
- Figure 3 is a perspective view of a sealed container, shown without its lid, containing a group of sterile syringes of the type illustrated in Figure 1;
- Figure 4 is a cross section of the container of Figure 3 in its closed configuration during a step in a preferred embodiment of the testing method according to the invention;
- Figures 5 and 6 show two different steps in another embodiment of the method according to the invention;
- Figure 7 is a schematic top plan view, with some parts cut away in order to better illustrate others, of a machine that implements the method of Figures 5 and 6;
- Figure 8 is a cross section through the broken line VIII-VIII of Figure 7;
- Figure 9 is an elevation view, with some parts in cross section, showing a detail of the machine of Figure 7.

### Detailed Description of the Preferred Embodiments of the Invention

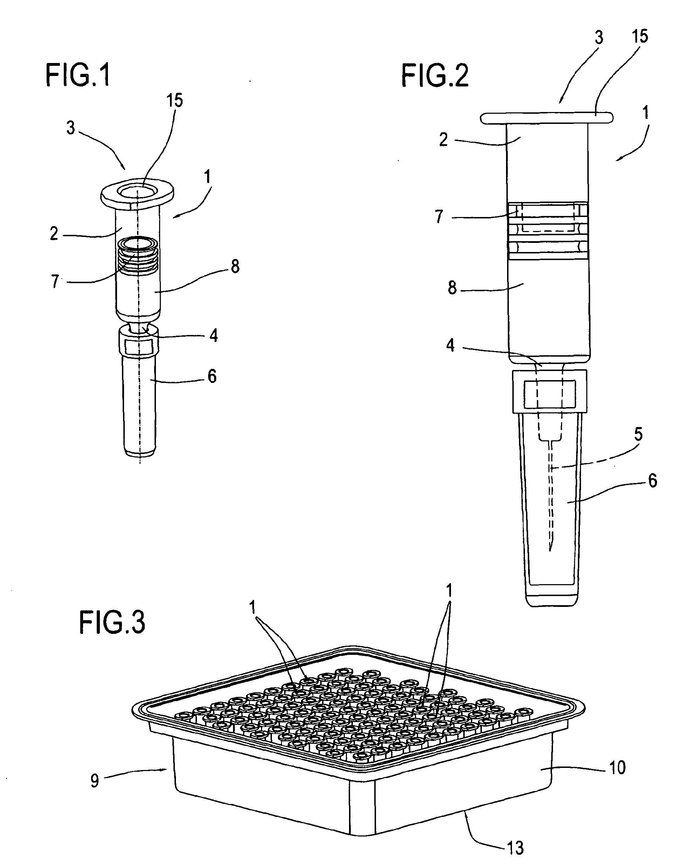

The numeral 1 in Figures 1 and 2 denotes in its entirety a sterile syringe of the disposable type prefilled with a medicinal product.

The syringe 1 comprises a hollow cylindrical barrel 2 for containing the medicinal product. One end 3 of the barrel 2 is open to enable it to be filled, while the other end is closed by a tip 4 to which a needle 5 is fitted and which is coupled with a cap 6 that protects the needle 5.

A rubber plunger 7 slides inside the barrel 2, forming a seal with the latter's inside surface and delimiting, at the end opposite the tip 4, a sterile airtight chamber 8 containing the medicinal product.

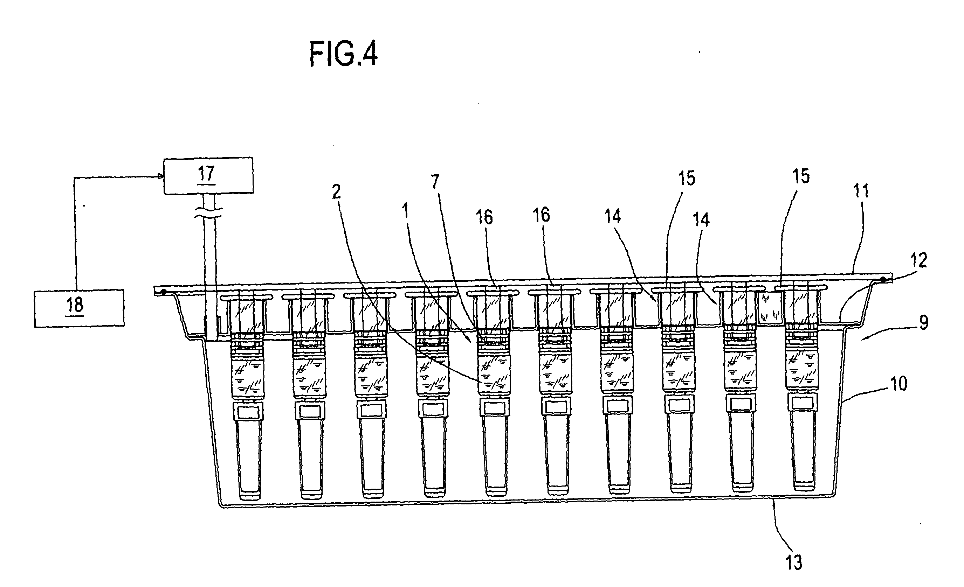

In a first embodiment of the invention, the syringe 1 is tested for leaks through the chamber 8 by placing the syringe 1, preferably together with a group of other syringes to be tested, in a container 9, illustrated in Figures 3 and 4.

The container 9 comprises a basin-shaped bottom body 10, a sealed lid 11 for hermetically closing the body 10 and a partition 12, parallel to a bottom wall 13 of the body 10.

The partition 12 has formed in it a plurality of cup-shaped cavities 14, arranged according to a defined distribution matrix and each designed to support a respective syringe 1 hanging perpendicularly to, and with the cap 6 at a predetermined distance from, the wall 13. A flange 15 formed on the end 3 of each syringe 1 rests on the upper edge of a respective cavity 14 for this purpose.

As illustrated in Figure 4, a plurality of rods 16, all equal in length, extend at right angles from the inside face of the lid 11. The rods 16 are arranged according to the same distribution matrix as the pockets 14 so that each faces a respective pocket 14 and is coaxial with the latter when the container 9 is closed.

Each rod 16 forms a stopping element which, when the container 9 is closed, is inserted into the barrel 2 of a respective syringe 1 and stops flush against the plunger 7 to prevent the latter from making even the smallest upward movement towards the outside of the barrel 2. In other words, by closing and opening the container 9, the rods 16 in their entirety constitute means for fixing and releasing the position of the plungers 7 relative to the barrels 2 of the respective syringes 1. For this purpose, the plunger 7 and the barrel 2 of each syringe 1 must be in exactly the same predetermined relative position as all the other syringes 1. This condition can be checked before closing the container 9 by measuring the positions of the plungers 7. These measured positions, as will become more apparent below, are then stored and used as initial positions with respect to a predetermined reference value. The measurement can be taken, for example, by one or more customary distance sensors, not illustrated.

The container 9 is then hermetically closed and a pressure different from atmospheric pressure is created inside it. In particular, a negative pressure is created inside the container 9 for a predetermined length of time using a suction source 17 controlled electronically by a control unit 18. More specifically, the internal pressure created is between 0.2 and 0.6 atmospheres, preferably 0.4 atmospheres, but it will be understood that the pressure may differ according to circumstances.

At this point, if each chamber 8, which contains the medicinal product at atmospheric pressure, is perfectly sealed, the negative pressure applied outside the syringes 1 does not have any effect inside the chamber 8 itself. At the same time, the rods 16 prevent the negative pressure from moving the plungers 7 upwards out of the barrels 2 of the respective syringes 1. When the pressure surrounding the syringes 1 is returned to atmospheric pressure and the container 9 is opened, the plungers 7 do not move from their original positions since the interior of each chamber 8 has remained at atmospheric pressure. This can be checked visually or, preferably, by measuring the final positions of the plungers 7 and comparing the final positions with the initial positions.

On the other hand, if a chamber 8 is not perfectly sealed, the pressure inside it is reduced by the negative pressure inside the container 9. When the pressure surrounding the syringes 1 is returned to atmospheric pressure, the plunger 7 moves from its initial position to a final position closer to the tip 4. This, as stated above, can be checked preferably by measuring the final positions of the plungers 7 and comparing the final positions with the initial positions. Evidently, however, the movement may also be observed by making a simple visual check on the syringes 1 subjected to the test described above.

In a second embodiment of the invention, a pump is used to raise the pressure inside the hermetically closed container 9 until it is higher than atmospheric pressure. In this case, instead of the rods 16, there are suction stems designed to prevent the plungers 7 from making even the smallest downward movement towards the inside of the barrels 2 and away from their initial positions.

In this embodiment, if a chamber 8 is not perfectly sealed, the pressure inside it is raised by the overpressure inside the container 9. When the pressure surrounding the syringes 1 is returned to atmospheric pressure and the container 9 is opened, the plunger 7 moves from its initial position to a final position further away from the tip 4. In this case, too, the movement of the plunger 7 can be easily detected in the same ways as those described above.

In a third embodiment of the invention, pressure lower than atmospheric pressure is applied to one part of each syringe 1 and higher than atmospheric pressure simultaneously to a different part of it. In this embodiment of the syringe 1 leak testing method, the position of the plunger 7 relative to a fixed reference in each syringe 1 is measured.

The syringe 1 is then placed in a testing head 19.

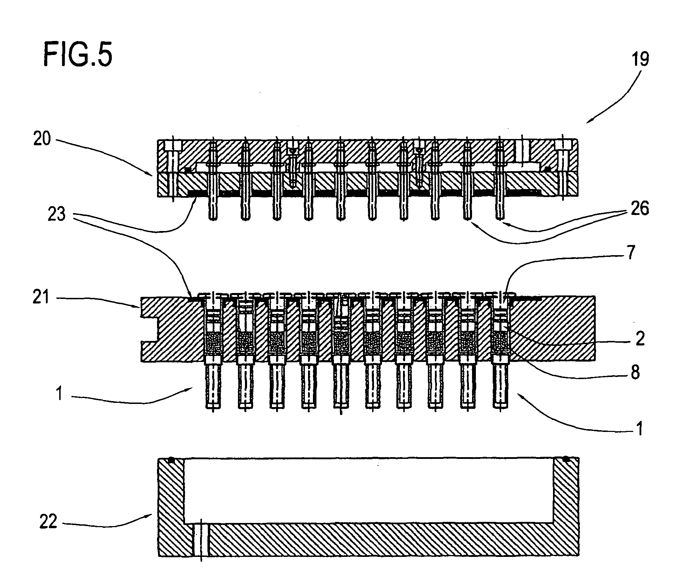

As illustrated in Figure 5, the testing head 19 comprises a first, upper mobile hood 20, a middle base plate 21, housing a plurality of syringes 1 and a second, lower mobile hood 22.

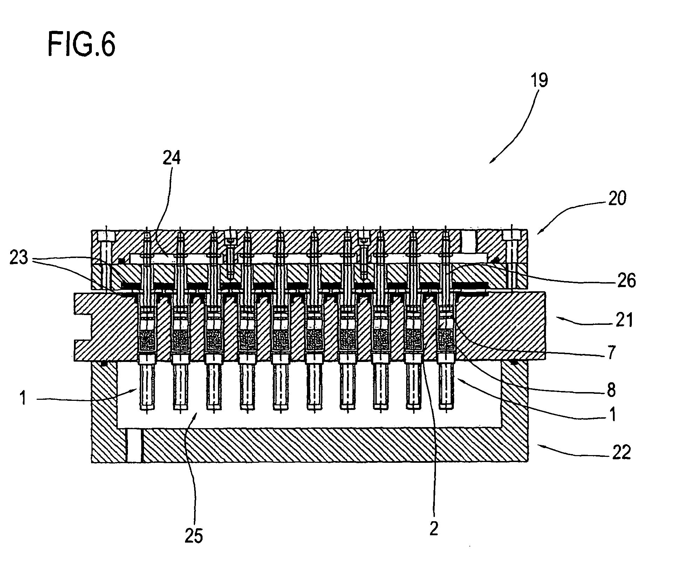

With reference to Figure 6, thanks to interposed hermetic seals 23, a first upper airtight chamber 24 is formed between the middle base plate 21 and the upper hood 20.

Similarly, a second lower airtight chamber 25 is formed between the middle base plate 21 and the lower hood 22.

The upper hood 20 has a plurality of stems 26 protruding downwards and designed to be inserted into the hollow cylindrical barrel 2 of each syringe 1, engaging the top face of the plunger 7, and capable of pushing the plunger 7 itself downwards for a certain length.

As illustrated in Figure 6, with the syringes 1 housed in the middle base plate 21, the upper hood 20 and the lower hood 22 are closed and tightened around the base plate 21 itself in such a way as to form the above mentioned upper airtight chamber 24 and lower airtight chamber 25.

Figure 6 also shows how the top end of the cylindrical barrel 2 is in communication with the first, upper chamber 24 while the outside surface of the chamber 8 and the tip 4 are in communication with the second, lower chamber 25.

Suitable suction means not illustrated are used to create a negative pressure, that is, pressure lower than atmospheric pressure, in the first, upper chamber 24, while a pump not illustrated simultaneously applies a pressure higher than atmospheric pressure in the second, lower chamber 25.

Under these conditions, the plunger 7 is exposed to the negative pressure in the upper chamber 24 but since it is held by a respective stem 26, it does not move from its position. If the plunger 7 is damaged or flawed so that the sterile chamber 8 is not hermetically sealed, the negative pressure extends into the chamber 8 itself and, when the upper hood 20 and the stems 26 are lifted, the plunger 7 remains in the lowered position.

The fact that the plunger 7, when returned to atmospheric pressure, does not move back to its initial position means that it does not provide an effective seal and, therefore, that the syringe 1 must be rejected.

As described above, the syringe 1 is also exposed to the overpressure created in the lower chamber 25. In the presence of cracks or flaws in the walls of the syringe 1 or in the tip 4, the overpressure extends into the chamber 8 and, when the upper hood 20 and the stems 26 are lifted and atmospheric pressure conditions restored, the plunger 7 not only returns to its position prior to being pushed down by the stem 26 but moves past that position and stops only when the pressure inside the chamber 8 is substantially the same as atmospheric pressure, that is to say, to a final position further away from the tip 4.

In this case, too, the fact that the plunger 7, when returned to atmospheric pressure, does not move back to its initial position means that the syringe 1 is defective and must be rejected.

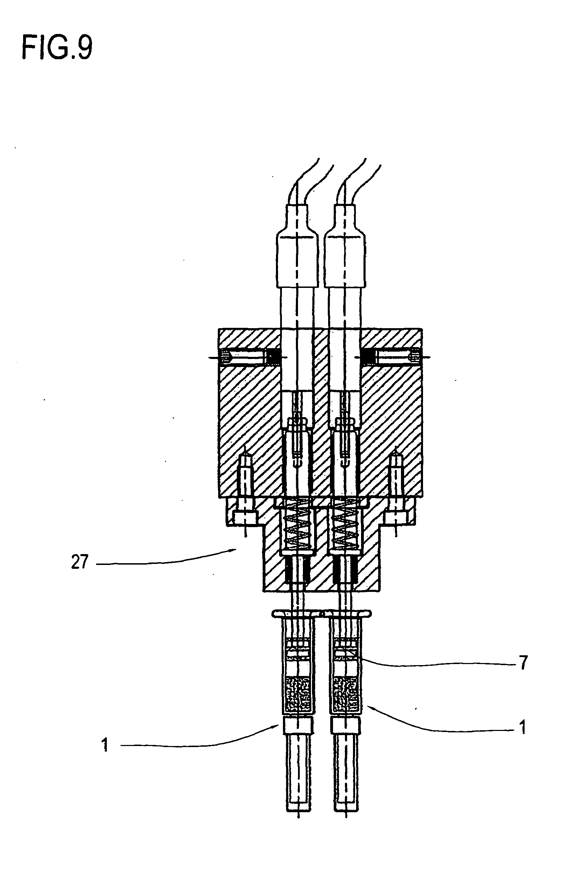

After the syringe 1 has been exposed simultaneously to negative pressure and overpressure (that is, after the testing step), the position of the plunger 7 is checked by a second set of detection elements 27 like the ones illustrated in Figure 9.

By comparing the positions detected for each plunger before and after the test, it is easy to see whether the plunger 7 has changed position after the syringe 1 has been simultaneously exposed to pressure below and above atmospheric pressure and, hence, whether the syringe 1 is hermetically sealed and thus leakproof or is not leakproof and must thus be rejected.

Advantageously, exposing the bottom of the syringe 1, that is, the outside surface of the barrel 2 and the tip 4, to a pressure higher than atmospheric pressure allows air to pass through even extremely small holes or cracks in the wall of the barrel 2. This makes it possible to locate microscopic leak sites in the syringe 1 that might not be otherwise detected if the same part were exposed to pressure below atmospheric pressure. That is because micro-cracks and other very small defects will allow air to leak through but not a liquid like the one in the syringe since the density of the liquid is greater than that of air.

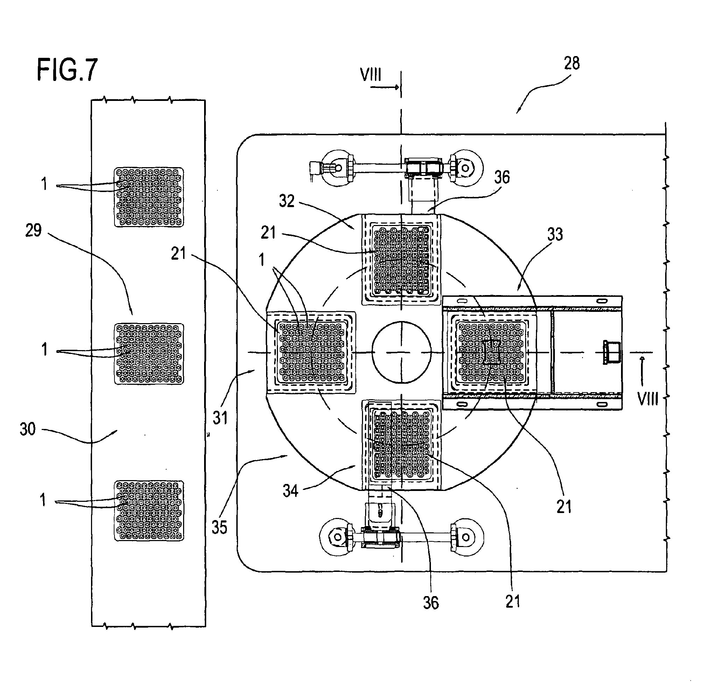

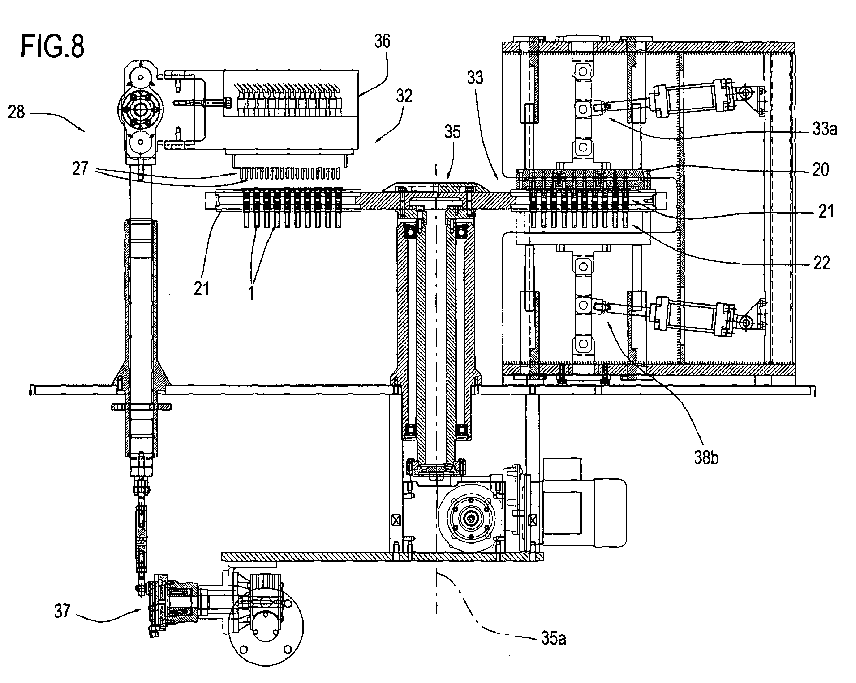

Figures 7 and 8 schematically illustrate a machine 28 designed to implement the third embodiment of the sterile syringe 1 leak testing method just described.

The syringes 1 to be tested, housed in suitable trays 29, are fed to the machine 28 by a conveyor belt 30.

The machine 28 comprises a syringe loading/unloading station 31, a first detecting station 32 for measuring the position of the plunger 7 of each syringe 1, a testing station 33 and a second detecting station 34 where the position of the plunger 7 is measured again.

Using suitable gripper means (not illustrated), the syringes 1 are picked up from the belt 30 and transferred to a dedicated housing base plate 21 at the loading/unloading station 31 of the machine 28. The machine 28 comprises four base plates 21, mounted at equal angular intervals on a carousel 35 rotatable about a respective axis of rotation 35a.

A rotation of the carousel 35 through 90° about its axis 35a, carries the base plate 21 that houses the syringes 1 to the first detecting station 32 where the position of the plunger 7 of each syringe 1 is detected and measured.

This detection step is performed by apparatus like that illustrated in Figure 9, whose detection elements 27 are moved into contact with the top surface of the plungers 7.

As illustrated in Figures 7 and 8, the detecting station 32 comprises a head 36 fitted with a plurality of detection elements 27, advantageously arranged in one or more rows. The detection elements 27 are driven in successive steps to measure the positions of the plungers 7 of all the syringes 1 in one base plate 21. More specifically, as illustrated in Figure 8, the head 36 is cyclically lifted and lowered in a direction parallel to the axis 35a, by a link and crank type mechanism 37.

When the first position detection step has been completed for all the syringes 1 on the base plate 21, the carousel 35 turns through a further 90° - clockwise with reference to Figure 7 - and moves the base plate 21 to the testing station 33.

At the testing station 33, toggle drive means 38a, 38b move the upper hood 20 and the lower hood 22 towards each other so as to tighten them to the base plate 21, as illustrated in Figure 6, in such a way as to form the above mentioned upper airtight chamber 24 and lower airtight chamber 25.

Under these conditions, negative pressure is applied to the upper chamber 24 and overpressure to the lower chamber 25, according to the method and for the purposes described above.

When the testing step has been completed, the carousel 35 turns through a further 90° - clockwise with reference to Figure 7 - and moves the base plate 21 to the second detection station 34.

The second detection station 34 performs the above mentioned second position detection during which, as described above with reference to the first detection station 32, the position of the plunger 7 of each syringe 1 is measured after the syringe 1 has been exposed to pressures different from atmospheric pressure during the testing step.

The detection elements 27 of the second detection station 34 are operated in the same way as described above with reference to the first detection station 32 and, for brevity, their operation will not be described again.

When the second position detection step has been completed for all the syringes 1 on the base plate 21, the carousel 35 turns through a further 90° - clockwise with reference to Figure 7 - and moves the base plate 21 to the loading/unloading station 31.

The above mentioned gripper means (not illustrated) pick up the syringes 1 from the base plate 21 positioned at the loading/unloading station 31 and transfer them to an empty tray 29 on the conveyor belt 30 which transports them to further processing units.

A computerized control unit, not illustrated, controls transfer operations, comparing the position measurements taken at the two detection stations 32, 34 and causing the defective syringes 1, found not to be leakproof, to be rejected.

The defective syringes 1 are picked up and expelled by gripper means (not illustrated) either before or after the syringes 1 are transferred to the conveyor belt 30.

## Claims

1. A leak testing method for sterile syringes, each comprising a barrel (2) for containing a medicinal product and a plunger (7) which slides inside the barrel (2) and forms a seal with the latter's inside surface, the method being **characterised in that** it comprises the steps of:
- fixing the position of the plunger (7) relative to the barrel (2) of each syringe (1) to be tested;
- exposing the syringe (1) to an outside pressure that is different from atmospheric pressure;
- exposing the syringe (1) to an outside pressure that is equal to atmospheric pressure;
- releasing the position of the plunger (7) relative to the barrel (2) of the syringe (1);
- detecting any change in the position of the plunger (7) relative to the barrel (2) of the syringe (1).

2. The method according to claim 1, **characterised in that**, after fixing the position of the plunger (7) relative to the barrel (2), the syringe (1) to be tested is exposed to an outside pressure that is lower than atmospheric pressure.

3. The method according to claim 2, **characterised in that** the outside pressure is between 0.2 and 0.6 atmospheres.

4. The method according to claim 3, **characterised in that** the outside pressure is 0.4 atmospheres.

5. The method according to any of the foregoing claims from 2 to 4, **characterised in that** the position of the plunger (7) relative to the barrel (2) of each syringe (1) to be tested is fixed by a stopping element (16) which stops flush against the plunger (7) on the outside of the syringe (1) to prevent the plunger (7) from moving upwards towards the outside of the barrel (2).

6. The method according to any of the foregoing claims from 1 to 5, **characterised in that** before fixing the position of the plunger (7) relative to the barrel (2) of each syringe (1) to be tested, said relative position is measured and used as initial position.

7. The method according to claim 6, **characterised in that** after resetting the pressure outside the syringe (1) to atmospheric pressure and releasing the position of the plunger (7) relative to the barrel (2) of each syringe (1) to be tested, said relative position is measured again and used as final position.

8. The method according to claim 7, **characterised in that** the initial and final positions are compared in order to detect any change in the position of the plunger (7) relative to the barrel (2) of the syringe (1) to be tested.

9. The method according to any of the foregoing claims from 1 to 8, **characterised in that** the syringes (1) are tested in a group inside a hermetically sealed container (9) comprising means (16) for fixing and releasing the position of the plunger (7) relative to the barrel (2) of each syringe (1) to be tested and within which a pressure lower than atmospheric pressure is created.

10. The method according to any of the foregoing claims from 1 to 8, **characterised in that** the step of exposing the syringe (1) to an outside pressure that is different from atmospheric pressure comprises a further step of exposing a first part (7) of the syringe to a pressure lower than atmospheric pressure and a further step of exposing a second part (2, 4) of the syringe (1) to a pressure higher than atmospheric pressure.

11. The method according to claim 10, **characterised in that** the two further steps are performed substantially simultaneously.

12. A leak testing machine for sterile syringes (1), each comprising a barrel (2) for containing a medicinal product and a plunger (7) which slides inside the barrel (2) and forms a seal with the latter's inside surface, the machine comprising:
a first detecting station (32) for measuring the position of the plunger (7) relative to the barrel (2);
a testing station (33) where the syringes (1) are exposed to outside pressures that are different from atmospheric pressure;
a second detecting station (34) for measuring the position of the plunger (7) relative to the barrel (2).

13. The machine according to claim 12, **characterised in that** it comprises a carousel (35) for transferring the syringes (1) between the stations (32, 33, 34).

14. The machine according to claim 12 or 13, where the syringes (1) are positioned on a respective housing base plate (21), **characterised in that** the testing station (33) comprises at least one upper hood (20) and one lower hood (22) designed to be closed and tightened around the base plate (21) in such a way as to form two airtight chambers (24, 25), respectively upper and lower.

15. The machine according to claim 14, **characterised in that** the upper hood (20) comprises a plurality of stems (26) designed to engage respective plungers (7) of the syringes (1) positioned on the housing base plate (21).

## Patentansprüche

1. Leckprüfverfahren für sterile Spritzen, jeweils bestehend aus einem Spritzenzylinder (2) zur Aufnahme eines Arzneimittels und einem Spritzenkolben (7), der in dem Zylinder (2) gleitet und abdichtend an dessen Innenwandung anliegt, wobei das Verfahren **dadurch gekennzeichnet ist, dass** es folgende Schritte beinhaltet:
- Fixieren der Position des Kolbens (7) relativ zu dem Zylinder (2) jeder zu prüfenden Spritze (1);
- Beaufschlagen der Spritze (1) mit einem Außendruck, der sich von dem atmosphärischen Druck unterscheidet;
- Beaufschlagen der Spritze (1) mit einem Außendruck, der gleich des atmosphärischen Drucks ist;
- Freigeben der Position des Kolbens (7) relativ zu dem Zylinder (2) der Spritze (1);
- Erfassen eventueller Änderungen der Position des Kolbens (7) relativ zu dem Zylinder (2) der Spritze (1).

2. Verfahren nach Anspruch 1, **dadurch gekennzeichnet, dass**, nach dem Fixieren der Position des Kolbens (7) relativ zu dem Zylinder (2), die zu prüfende Spritze (1) mit einem Außendruck beaufschlagt wird, der niedriger ist als der atmosphärische Druck.

3. Verfahren nach Anspruch 2, **dadurch gekennzeichnet, dass** der Außendruck zwischen 0,2 und 0,6 Atmosphären beträgt.

4. Verfahren nach Anspruch 3, **dadurch gekennzeichnet, dass** der Außendruck 0,4 Atmosphären beträgt.

5. Verfahren nach einem der vorhergehenden Ansprüche von 2 bis 4, **dadurch gekennzeichnet, dass** die Position des Kolbens (7) relativ zu dem Zylinder (2) jeder zu prüfenden Spritze (1) durch ein Stopperelement (16) fixiert wird, das an der Außenseite der Spritze (1) bündig gegen den Kolben (7) anliegt, um zu verhindern, dass sich der Kolben (7) nach oben aus dem Zylinder (2) heraus bewegen kann.

6. Verfahren nach einem der vorhergehenden Ansprüche von 1 bis 5, **dadurch gekennzeichnet, dass** vor dem Fixieren der Position des Kolbens (7) relativ zu dem Zylinder (2) jeder zu prüfenden Spritze (1) diese Relativposition gemessen und als Ausgangsposition verwendet wird.

7. Verfahren nach Anspruch 6, **dadurch gekennzeichnet, dass** nach der Wiedereinstellung des Drucks außerhalb der Spritze (1) auf den atmosphärischen Druck und Freigeben der Position des Kolbens (7) relativ zu dem Zylinder (2) jeder zu prüfenden Spritze (1) diese Relativposition erneut gemessen und als Endposition verwendet wird.

8. Verfahren nach Anspruch 7, **dadurch gekennzeichnet, dass** die Ausgangs- und Endpositionen miteinander verglichen werden, um eventuelle Änderungen der Position des Kolbens (7) relativ zu dem Zylinder (2) der zu prüfenden Spritze (1) zu erfassen.

9. Verfahren nach einem der vorhergehenden Ansprüche von 1 bis 8, **dadurch gekennzeichnet, dass** die Spritzen (1) gruppenweise im Innern eines hermetisch geschlossenen Behälters (9) geprüft werden, der Mittel (16) für das Fixieren und Freigeben der Position des Kolbens (7) relativ zu dem Zylinder (2) jeder zu prüfenden Spritze (1) beinhaltet und innerhalb dessen ein Druck erzeugt wird, der niedriger ist als der atmosphärische Druck.

10. Verfahren nach einem der vorhergehenden Ansprüche von 1 bis 8, **dadurch gekennzeichnet, dass** der Schritt des Beaufschlagens der Spritze (1) mit einem Außendruck, der sich von dem atmosphärischen Druck unterscheidet, weiter einen Schritt beinhaltet, bei dem ein erster Teil (7) der Spritze einem niedrigeren Druck als dem atmosphärischen Druck ausgesetzt wird, und weiter einen Schritt, bei dem ein zweiter Teil (2, 4) der Spritze (1) einem höheren Druck als dem atmosphärischen Druck ausgesetzt wird.

11. Verfahren nach Anspruch 10, **dadurch gekennzeichnet, dass** die zwei weiteren Schritte im Wesentlichen gleichzeitig ausgeführt werden.

12. Leckprüfmaschine für sterile Spritzen (1), jeweils bestehend aus einem Spritzenzylinder (2) zur Aufnahme eines Arzneimittels und einem Spritzenkolben (7), der in dem Zylinder (2) gleitet und abdichtend an dessen Innenwandung anliegt, wobei die Maschine Folgendes beinhaltet:
eine erste Erfassungsstation (32) zum Messen der Position des Kolbens (7) relativ zu dem Zylinder (2);
eine Prüfstation (33), in der die Spritzen (1) Außendruckwerten ausgesetzt werden, die sich von dem atmosphärischen Druck unterscheiden;
eine zweite Erfassungsstation (34) zum Messen der Position des Kolbens (7) relativ zu dem Zylinder (2).

13. Maschine nach Anspruch 12, **dadurch gekennzeichnet, dass** sie einen Rundläufer (35) zur Beförderung der Spritzen (1) zwischen den Stationen (32, 33, 34) beinhaltet.

14. Maschine nach Anspruch 12 oder 13, worin die Spritzen (1) auf einer entsprechenden Aufnahmegrundplatte (21) angeordnet werden, **dadurch gekennzeichnet, dass** die Prüfstation (33) zumindest eine obere Haube (20) und eine untere Haube (22) beinhaltet, die dafür vorgesehen sind, um die Grundplatte (21) herum geschlossen und abgedichtet zu werden, so dass zwei luftdichte Kammern (24, 25) gebildet werden, genauer gesagt eine obere und eine untere Kammer.

15. Maschine nach Anspruch 14, **dadurch gekennzeichnet, dass** die obere Haube (20) mehrere Schäfte (26) beinhaltet, die dafür ausgelegt sind, in Eingriff mit entsprechenden Kolben (7) der auf der Aufnahmegrundplatte (21) angeordneten Spritzen (1) zu gelangen.

## Revendications

1. Un procédé de test de fuite de seringues stériles comprenant, chacune, un corps (2) destiné à contenir un produit médicinal et un piston (7) qui coulisse à l'intérieur dudit corps (2) et forme un joint d'étanchéité avec la surface intérieure de ce dernier, tel procédé étant **caractérisé en ce qu'**il comprend les phases consistant à :
- bloquer la position du piston (7) par rapport au corps (2) de chaque seringue (1) à tester ;
- soumettre la seringue (1) à une pression extérieure autre que la pression atmosphérique ;
- soumettre la seringue (1) à une pression extérieure égale à la pression atmosphérique ;
- débloquer la position du piston (7) par rapport au corps (2) de la seringue (1) ;
- détecter une éventuelle variation de la position du piston (7) par rapport au corps (2) de la seringue (1).

2. Le procédé selon la revendication 1, **caractérisé en ce que** la seringue (1) à tester est soumise, après blocage de la position du piston (7) par rapport au corps (2), à une pression extérieure qui est inférieure à la pression atmosphérique.

3. Le procédé selon la revendication 2, **caractérisé en ce que** la pression extérieure est comprise entre 0,2 et 0,6 atmosphère.

4. Le procédé selon la revendication 3, **caractérisé en ce que** la pression extérieure est égale à 0,4 atmosphère.

5. Le procédé selon l'une quelconque des revendications précédentes de 2 à 4, **caractérisé en ce que** la position du piston (7) par rapport au corps (2) de chaque seringue
(1) à tester est bloquée au moyen d'un élément d'arrêt (16) qui vient en butée contre le piston (7) à l'extérieur de la seringue (1) pour empêcher tout mouvement vers le haut dudit piston (7) vers l'extérieur du corps (2).

6. Le procédé selon l'une quelconque des revendications précédentes de 1 à 5, **caractérisé en ce que**, avant de bloquer la position du piston (7) par rapport au corps (2) de chaque seringue (1) à tester, ladite position relative est mesurée et utilisée comme position initiale.

7. Le procédé selon la revendication 6, **caractérisé en ce que**, après avoir ramené la pression extérieure à la seringue (1) à la pression atmosphérique et avoir débloqué la position du piston (7) par rapport au corps (2) de chaque seringue (1) à tester, ladite position relative est à nouveau mesurée et utilisée comme position finale.

8. Le procédé selon la revendication 7, **caractérisé en ce que** les positions initiale et finale sont comparées afin de détecter une éventuelle variation de la position du piston (7) par rapport au corps (2) de la seringue (1) à tester.

9. Le procédé selon l'une quelconque des revendications précédentes de 1 à 8, **caractérisé en ce que** les seringues (1) sont testées en groupe à l'intérieur d'un contenant (9) hermétiquement étanche comprenant des moyens (16) servant à bloquer et à débloquer la position du piston (7) par rapport au corps (2) de chaque seringue (1) à tester et à l'intérieur duquel une pression inférieure à la pression atmosphérique est générée.

10. Le procédé selon l'une quelconque des revendications précédentes de 1 à 8, **caractérisé en ce que** la phase consistant à soumettre la seringue (1) à une pression extérieure autre que la pression atmosphérique comprend une autre phase consistant à soumettre une première partie (7) de la seringue à une pression inférieure à la pression atmosphérique et une autre phase consistant à soumettre une deuxième partie (2, 4) de la seringue (1) à une pression supérieure à la pression atmosphérique.

11. Le procédé selon la revendication 10, **caractérisé en ce que** lesdites deux autres phases sont effectuées essentiellement en même temps.

12. Un dispositif de test de fuite de seringues stériles (1) comprenant, chacune, un corps (2) destiné à contenir un produit médicinal et un piston (7) qui coulisse à l'intérieur dudit corps (2) et forme un joint d'étanchéité avec la surface intérieure de ce dernier, tel dispositif comprenant :
une première station de détection (32) servant à mesurer la position du piston (7) par rapport au corps (2) ;
une station de test (33) où les seringues (1) sont soumises à des pressions extérieures autres que la pression atmosphérique ;
une deuxième station de détection (34) servant à mesurer la position du piston (7) par rapport au corps (2).

13. Le dispositif selon la revendication 12, **caractérisé en ce qu'**il comprend un carrousel (35) servant à transférer les seringues (1) entre les stations (32, 33, 34).

14. Le dispositif selon la revendication 12 ou 13, où les seringues (1) sont positionnées sur une plaque de base (21) respective de logement, **caractérisé en ce que** la station de test (33) comprend au moins une hotte supérieure (20) et une hotte inférieure (22) destinées à être fermées hermétiquement autour de la plaque de base (21) de manière à former deux chambres (24, 25) étanches à l'air, respectivement supérieure et inférieure.

15. Le dispositif selon la revendication 14, **caractérisé en ce que** la hotte supérieure (20) comprend une pluralité de tiges (26) destinées à assujettir des pistons (7) respectifs appartenant aux seringues (1) positionnées sur la plaque de base (21) de logement.
